# EUROPEAN PATENT APPLICATION

(11) **EP 2 404 617 A1**
(43) Date of publication of application: **11.01.2012**
(21) Application number: 10425231.7
(22) Date of filing: 08.07.2010
(51) Int. Cl.: A61K 41/00, A61P 43/00

(54) **Frequency physical carrier for diagnostics, medical therapy and human, zootechnical and agronomic enhancement**

(71) Applicant: Spera, Dario Maximilian, 00147 Roma (IT)
(72) Inventor: Spera, Dario Maximilian, 00147 Roma (IT)
(74) Representative: Cioncoloni, Giuliana

(57) **Abstract**

Physical carrier wherein electromagnetic frequencies are recorded, obtained by the following process:
*a)* generating biologically active radiation frequencies, including frequencies of electromagnetic radiation, including optical radiation, and including frequencies of mechanical radiation, including acoustic radiation, which frequencies are synthesized or sensed by biologically active chemical compounds, including medicaments; hormones, including prolactin; parasiticides; antiseptics;
*b)* loading said biologically active frequencies into the store of a computer;
*c)* digital processing of said biologically active frequencies loaded into said store of a computer, implemented by a digital data processing programme, including programmes for processing photographs, videos, audios or texts.

## Description

This invention relates to a physical carrier configured as a data store which is non-volatile (i.e. able to keep stored data and to make these ones available when it is not electrically supplied).

It is known that in medical diagnostics laboratory analyses are often carried out that require some time and at times the use becomes necessary of costly machineries not always available at the moment. Moreover, it is also known that in medical therapy and for the enhancement of human performances one makes use of chemical compounds. For zootechnical and agronomic enhancement too, such as e.g. for the storing of food, or the stimulation of the production of milk, one makes use of chemical compounds, particularly hormones and parasiticides. However, well known disadvantages and restrictions are connected to the chemical action of the latter.

This invention has the object of providing diagnostic, therapeutic means and means for human, zootechnical and agronomic enhancement that achieve the effects of biologically active chemical compounds, without the disadvantages and the restrictions of chemical action (by the expression "biologically active" those chemical compounds are meant herein that have an effect on biologic tissues, particularly those of medical, both diagnostic and therapeutic; of human enhancement interest; of zootechnical and agronomic usefulness).

The studies are known of Nobel Prize for Medicine Luc Montagnier who has put into evidence that in living organisms the appearance of specific diseases corresponds to specific deformations of the electromagnetic structure of the organism and that there are electromagnetic signals specific for each disease.

International Application No. WO 94/17406, having the title "PROCEDE ET DISPOSITIF DE TRASMISSION SOUS FORME DE. SIGNAL DE L'ACTIVITE BIOLOGIQUE D'UNE MATIERE PORTEUSE A UNE AUTRE MATIERE PORTEUSE, ET DE TRAITEMENT D'UN TEL SIGNAL, ET PRODUIT OBTENU AVEC UN TEL PROCEDE" (Inventor Jacques BENVENISTE), discloses a method and a device used to transmit the biological activity of a first matter said the carrier to a second matter said the target. The method consists in exposing the biological activity carrier to a detector of electromagnetic signals; amplifying the emitted electromagnetic signals characteristic of the manifestation of the biological activity and thereafter exposing the target to an electromagnetic signal emitter connected to the detector through a transmission and amplification circuit, in order to transmit the signal characteristic of the biological activity of the target.

International Application No. WO 94/17637, having the title "PROCEDE ET SYSTEME POUR PRODUIRE UNE SUBSTANCE OU UN SIGNAL AYANT UN EFFET COAGULANT OU ANTICOAGULANT" (inventors Jacques BENVENISTE and Didier GUILLONNET), discloses a method and a system for recording and digitize an electrical signal characteristic of a molecule possessing a biological activity, after analogue-to-digital conversion by the aid of a computer.

International Application No. WO 2007/068831, having the title "METHOD FOR CHARACTERISING A BIOLOGICALLY ACTIVE BIOCHEMICAL ELEMENT BY ANALYSING LOW FREQUENCY ELECTROMAGNETIC SIGNALS" (inventor Luc MONTAGNIER), discloses a method for characterizing a biologically active biochemical element through the analysis of low frequency electromagnetic signals emitted by a sample of analyzable material.

International Application No. WO 2007/147982, having the title "METHOD OF DETECTING MICROORGANISMS WITHIN A SPECIMEN" (inventors Luc MONTAGNIER and Aissa JAMAL), discloses a method for the diagnostic detection of microorganisms through the frequencies of the latter.

Therefore, this invention reaches the object above by exploiting the action of the electromagnetic frequencies of biologically active chemical compounds. The frequencies are recorded i.e. downloaded in a physical carrier capable of keeping them in a non-volatile way, i.e. without needing for a supply of electric power, and of re-emitting them coming in contact with another material carrier because of potential difference with respect to the latter. The other material carrier may be human skin.

Therefore, it is the subject-matter of this invention a carrier according to annexed Claim 1.

Preferred embodiments are set forth in dependent claims.

This invention will be understood based on the following exemplifying disclosure, absolutely not restrictive thereof, having reference to specific embodiments thereof.

This invention is a physical carrier wherein electromagnetic frequencies are recorded, obtained by the following process:
*a)* generating biologically active radiation frequencies, including electromagnetic radiation frequencies, including optical radiation, and including mechanical radiation frequencies, including acoustic radiation, which frequencies are synthesized or sensed by biologically active chemical compounds, including medicaments; hormones, including prolactin; parasiticides; antiseptics;
*b)* loading said biologically active frequencies into the store of a computer;
*c)* digital processing of said biologically active frequencies loaded into said store of a computer, implemented by a digital data processing programme, including programmes for processing photographs, videos, audios or texts.

The frequencies useful for the inventive purposes may be the electromagnetic frequencies emitted by medicaments as can be senses through an apparatus for sensing electromagnetic frequencies, such as e.g. apparatus "Vega-Test". This one is an electromedical apparatus comprising a Wheatstone bridge circuit containing a galvanometer having a reading dial, a direct current source and a metal honeycomb casket in which medicaments may be inserted in series to the circuit. "Vega-Test" apparatus is capable of recording the electromagnetic frequencies of substances in general, and of medicaments in particular, through a process of analogue-to-digital conversion, of saving these ones in a store and subsequently of re-emitting them re-transforming them into electromagnetic signals through a digital-to-analogue conversion process. The apparatus is intended to be used in cooperation with an electrode, which a patient keeps in his/her hand, and a tip which is put in contact with the skin of the patient, at an elective point, so a patient-apparatus circuit being closed. Having inserted a determined substance in the apparatus, the response is read on the dial to the substance and to the characteristic frequency of the substance.

The electromagnetic frequencies e.g. of a medicament may also be sensed by means of a high resolution camera, possibly also sensible to infrared and ultraviolet radiation.

The frequencies useful for the inventive purposes may be obtained with synthesizers. The frequencies suitable for determining a therapeutic or human enhancement effect are determined based on experimental tests, evaluating the response of human subjects to inventive carriers that have data recorded therein obtained starting from determined frequencies. In particular one may evaluate the responses to specific kinesiologic tests, or tests on stabilometric platform to evaluate the betterment of equilibrium.

It is envisaged according to this invention that the digital processing of the frequencies may be carried out with a programme for rendering input data into image format data. Photograph management and processing programmes may be used, such as e.g. Adobe Photoshop. In particular, through the function "Transparency" of such programmes it is possible to 'dilute' i.e. to partialize the effect of the electromagnetic frequencies on the human body. For instance, in case the frequencies are the electromagnetic frequencies of a medicament, it is so possible with the inventive carrier to emulate the administration of half a tablet of the medicament.

The digital processing of the frequencies may, on the other hand, be carried out with a processing programme in video, audio or text format, and more generally with any suitable digital processing programme.

The inventive carrier may be a solid state microelectronic circuit i.e. a microchip.

The inventive carrier may also be any store medium allowing the passage of frequencies onto any useful material, such as a magnetic store, an optical store, a holographic store.

Moreover, the inventive carrier may be composed of a plastic material, such as polyethylene (PE); polyethylene terephthalate (PET); polypropylene (PP); polyvinylchloride (PVC); polyvinyl methacrylate (PVMA); polymethyl methacrylate (PMMA); polyamide (PA) or Nylon; polyether ketone (PEK); polytetrafluoroethylene (PTFE); polycarbonate (PC) or polyesters of carbonic acid; and copolymers thereof.

The inventive carrier still further may be composed of an elastomer, such as polybutadiene rubber (PBR); natural rubber (NR) i.e. 1-4 polyisoprene; chloroprene rubber (CR) i.e. polychloroprene; silicone rubbers, such as methyl vinyl polysiloxane (VMO); fluoro elastomers and copolymers thereof.

The inventive carrier composed of a plastic material or an elastomer may be in the shape of a film, in particular a strip. This one may be in particular in the shape of a bracelet. The strip of useful material may be 'activated' by impressing the same with the frequencies in digital format also downloading the same thereinto from a microchip. The useful material of the strip may be selected among those which are retentive enough not to lose the impressed i.e. recorded frequencies. So, for instance, a strip may be stored in a container without deactivating. The frequencies may only be removed from a strip through erasing, for instance to impress the strip with new frequencies. The frequency passage from the microchip to the useful material takes place through materials conducting said frequencies such as silicates and water. The frequencies are impressed by arranging the useful materials inside a Faraday cage put under voltage, so the frequencies amplified, these ones can be impressed in all of the material arranged inside the cage.

The inventive carrier may also be composed of a siliceous material i.e. a material substantially composed of silicates, such as quartz; quartz glass; glass (substantially composed of sodium and calcium silicate); lead glass (substantially composed of siliceous glass containing lead oxide in addition to potassium and sodium oxides); steatite (a rock substantially composed of hydrated magnesium silicate); calcareous rock, including travertine silicate; natural and synthetic crystals and stones.

More generally, the inventive carrier may be composed of any material useful for recording and emitting frequencies downloaded therein.

Putting the inventive carrier in physical contact with the epidermis of a human subject at elective points an electric potential difference is determined between the carrier and the epidermis, that activates the emission of electromagnetic frequencies on the part of the carrier itself.

The electromagnetic frequencies emitted by the inventive carrier act on the body of the subject performing a therapeutic action (like a chemical medicament) or an action of performance enhancement. For instance, with the inventive carrier it is possible to better the postural equilibrium of the body substantially.

It is also envisaged that the inventive carrier is realized in the shape of a grid wherein electromagnetic frequencies of parasiticides have been downloaded. Such a grid arranged in a water stream is suitable for conditioning the water itself with the frequencies of the parasiticides, with benefit for the cattle which will take such frequencies by drinking the water.

Analogously an inventive carrier may have the frequency of prolactin recorded therein to stimulate the production of milk.

This invention has been disclosed having reference to exemplifying embodiments thereof, but variations may be made thereto without departing from the scope of protection thereof, which only is defined by the appended claims.

### Bibliography

- F.A. Popp et al., "Electromagnetic Bio-Information", Urban & Schwarzenberg Verlag, 1989;
- H.W. Schimmel et al., "Short Manual of the Vega Test Method Bioenergetic Regulatory Technique", Vega Grieshaber, Schiltach, 1981;
- H.W. Schimmel et al., "Manuale del Metodo Vegatest", Ed. Named;
- Luc Montagnier, Convention on "Integration between physics, chemistry and biology at the basis of the medicine of the future", held in Milan on 30th September, 2009.

## Claims

1. Physical carrier wherein electromagnetic frequencies are downloaded i.e. recorded, obtained by the following process:
a) generating biologically active radiation frequencies, including electromagnetic radiation frequencies, including optical radiation, and including mechanical radiation frequencies, including acoustic radiation, which frequencies are synthesized or sensed by biologically active chemical compounds, including medicaments; hormones, including prolactin; parasiticides; antiseptics;
*b)* loading said biologically active frequencies into the store of a computer;
*c)* digital processing of said biologically active frequencies loaded into said store of a computer, implemented by a digital data processing programme, including programmes for processing photographs, videos, audios or texts.

2. Carrier of Claim 1, composed of a solid state microelectronic circuit or microchip.

3. Carrier of Claim 1, composed of a store medium allowing the passage of frequencies onto any useful material, including a magnetic store, an optical store, a holographic store.

4. Carrier of Claim 1, composed of a plastic material, including polyethylene (PE); polyethylene terephthalate (PET); polypropylene (PP); polyvinylchloride (PVC); polyvinyl methacrylate (PVMA); polymethyl methacrylate (PMMA); polyamide (PA) or Nylon; polyetherketone (PEK); polytetrafluoroethylene (PTFE); polycarbonate (PC) or polyesters of carbonic acid; and copolymers thereof.

5. Carrier of Claim 4, in the shape of a film, including a strip.

6. Carrier of Claim 1, composed of an elastomer, including polybutadiene rubber (PBR); natural rubber (NR) i.e. 1-4 polyisoprene; chloroprene rubber (CR) i.e. polychloroprene; silicone rubbers, including methylvinyl-polysiloxane (VMO); fluoroelastomers and copolymers thereof.

7. Carrier of Claim 6, in the shape of a film, including a strip.

8. Carrier of Claim 1, composed of a siliceous material, including quartz; quartz glass; glass; lead glass; steatite; calcareous rock, including travertine silicate; natural and synthetic crystals and stones.

9. Carrier of Claim 1 in the shape of a grid.

10. Carrier of Claim 1 composed of any material useful for recording and emitting frequencies downloaded therein.
